Europäisches Patentamt

European Patent Office

Office européen des brevets

(11) Publication number: **0 172 914**
**B1**

(12) **EUROPEAN PATENT SPECIFICATION**

(45) Date of publication of patent specification: 16.05.90

(21) Application number: 85900771.8

(22) Date of filing: 08.02.85

(86) International application number:
PCT/JP85/00053

(87) International publication number:
WO 85/03519 15.08.85 Gazette 85/18

(51) Int. Cl.⁵: **C 12 N 1/20**, A 01 G 13/00,
A 01 N 63/00 // (C12N1/20,
C12R1:38)

(54) PSEUDOMONAS SOLANACEARUM M4S STRAIN, COMPOSITION CONTAINING THE SAME FOR CONTROLLING SOIL-BORNE DISEASES OF SOLANACEOUS PLANT, AND METHOD FOR CONTROLLING SAID SOIL-BORNE DISEASES WITH THE SAME.

(30) Priority: 10.02.84 JP 21912/84

(43) Date of publication of application:
05.03.86 Bulletin 86/10

(45) Publication of the grant of the patent:
16.05.90 Bulletin 90/20

(84) Designated Contracting States:
FR GB

(56) References cited:

CHEMICAL ABSTRACTS, vol. 82, no. 13, March 31, 1975, page 268, Columbus, Ohio, US; W.G. RATHMELL et al.: "Induced resistance in tobacco leaves. Role of inhibitors of bacterial growth in the intercellular fluid"; & PHYSIOL. PLANT. PATHOL.. 1975, 5(1), 65-73

The file contains technical information submitted after the application was filed and not included in this specification

(73) Proprietor: Japan Tobacco Inc.
2-1 Toranomon, 2-Chome
Minato-Ku Tokyo 105 (JP)

(72) Inventor: TANAKA, Hiroshi 5116-3, Ohaza Tomonuma
Nogimachi, Shimotsukagun
Tochigi-ken 329-01 (JP)
Inventor: NAKAZAWA, Kunio
11-11, Wakagicho 2-chome
Koyama-shi Tochigi-ken 323 (JP)

(74) Representative: Popp, Eugen, Dr. et al
MEISSNER, BOLTE & PARTNER
Widenmayerstrasse 48 Postfach 86 06 24
D-8000 München 86 (DE)

(56) References cited:

Ann. Phytopath. Soc. Japan, Vol. 49, No. 1 (January.1983) Hiroshi Tanaka (Protection of Tobacco against Root Intection of Pseudomonas solanacearum by Heat-Killed Bacterial Cells) P. 66-68

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

## Description

### Technical Field

The present invention relates to a new micro-organism strain, a composition containing it for the control of soil-borne diseases, and a process using it for the control of bacterial wilt of tobacco and other solanaceous plants.

### Background Art

Bacterial wilt of tobacco and other solanaceous plants, such as eggplants, tomatoes, green peppers or the like, and brown rot of potatoes, are caused by *Pseudomonas solanacearum*. If infected with this disease, the plants wilt and yield poor harvest, resulting in a large loss.

As one of control measures for bacterial wilt of tobacco and other solanaceous plants, the present inventors have already filed a patent application regarding a process for controlling soil-borne diseases of solanaceous plants by applying heat-killed *Pseudomonas solanacearum* M23R (hereinafter referred to merely as "M23R") to the soil of plant roots (Japanese Patent Application No. 57—103982).

A report from the U.S.A. (W. Chen, E. Echandi & H. W. Spurr, Jr., Protection of Tobacco Plants from Bacterial Wilt with Avirulent Bacteriocin-Producing Strains, in Proc. Fifth Int. Con. Plant Bact. Coli, 1981, pp. 482—492) exists regarding a case in which the occurrence of bacterial wilt of tobacco was effectively controlled by inoculating tobacco roots with living cells of a mutant strain induced from a virulent strain of *Pseudomonas solanacearum*. In the report, the strain claimed to provide the most effective control for the bacterial wilt of tobacco was *Pseudomonas solanacearum* 305 (hereinafter referred to merely as "305 strain").

### Disclosure of Invention

It is an object of the present invention to provide a new bacterial strain having superior control effects on bacterial wilt of tobacco and other solanaceous plants to conventional strains, a composition containing the bacterial strain for control of bacterial wilt of tobacco and other solanaceous plants including brown rot of potato, and a process using the bacterial strain for the control of bacterial wilt of tobacco and other solanaceous plants.

The present invention provides a *Pseudomonas solanacearum* M4S strain.

The present invention also provides a composition for control of bacterial wilt of tobacco and other solanaceous plants, containing living cells of the *Pseudomonas solanacearum* M4S strain, and a pesticidally acceptable carrier.

The present invention further provides a process for control of bacterial wilt of tobacco and other solanaceous plants by inoculating plant roots with living cells of the *Pseudomonas solanacearum* M4S strain.

According to the present invention, there is provided a composition, containing a new bacterial strain —*Pseudomonas solanacearum* M4S—and its living cells, which has excellent control effects on soil-borne diseases of bacterial wilt of tobacco and other solanaceous plants.

The process according to the present invention controls bacterial wilt of tobacco and other solanaceous plants in a highly effective manner. The *Pseudomonas solanacearum* M4S strain is completely avirulent and acts merely on designated crops, causing no damage to the environment.

### Best Mode of Carrying Out the Invention

The new bacterial strain provided by the present invention is the *Pseudomonas solanacearum* M4S strain. This strain is obtained by mutation from a virulent *Pseudomonas solanacearum* by procedures described in detail hereinafter. The strain was initially deposited as FERM P-7370 at the Fermentation Research Institute, Agency of Industrial Science and Technology at 1—3, 1-chome, Yatabe-cho Higashi, Tsukuba-gun, Ibaraki-ken, Japan, on December 14, 1983, and was again deposited at the same location on January 24, 1985 as FERM BP-700, on the basis of the Budapest Treaty on the International Recognition of the Deposit of Microorganisms for the Purposes of Patent Procedure.

The *Pseudomonas solanacearum* M4S has the following bacteriological properties:

I. Morphological properties:
Gram-negative, rods, motile with 1 to 4 flagella of about 0.5 to 0.8 μm × 0.9 to 2.0 μm

II. Characteristics on culture media:
TZC plate medium (1 g of casamino acid, 10 g of peptone, 10 g of glucose, 18 g of agar, 1 liter of water; with 0.005% of 2,3,5-triphenyltetrazolium chloride as described in Kelman, A. Phytopathology, Vol. 44, pp. 693 to 695 (1954)). Small afluidal colonies formed.

A brown pigment is slightly produced on the agar medium containing tyrosine.

III. Physiological properties:
Aerobic

| Growing temperatures: | 10 to 38°C |
| Optimum growing temperatures: | 28 to 30°C |
| Nitrite formation reaction: | positive |
| Decomposition of starch: | negative |
| Decomposition of gelatin: | negative |
| Growing pH: | 4.3 to 9.0 |

IV. Utilizable carbon sources:
Lactose, glucose, fructose, sucrose, gluconic acid, p-hydroxybenzoic acid.
Non-utilizable carbon sources:
Arabinose, malonic acid, ethanol, testosterone.

These properties are identical to those of *Pseudomonas solanacearum* described in Buchanan, R. E. & Gibbons, N. E. Bergey's Manual of Deterministic Bacteriology, 8th Edition, pp. 231 to 233, and this organism is determined as belonging to this species.

However, it should be noted that *Pseudomonas solanacearum* M4S differs in bacteriological properties from the parent strain *Pseudomonas solanacearum*, described in the above literture, in terms of the formation of small afluidal colonies (the parent strain forms fluid colonies), and the production of a substance inhibiting the growth of the parent strain in the medium (the parent strain produces no substance inhibiting the growth of itself).

The composition used for control of soil-borne diseases according to the present invention contains living cells of the *Pseudomonas solanacearum* M4S strain described above, and a pesticidally acceptable carrier. The pesticidally acceptable carrier is widely known in the art, so that it is not necessary to describe it in detail here, except to say that water is preferred. Indeed the most preferred composition in the present invention is a suspension of the *Pseudomonas solanacearum* M4S strain in water. Preferred concentrations of the strain may range from $10^6$ to $10^{10}$ cells/ml, and, most preferably from $10^7$ to $10^9$ cells/ml.

The process for the control of soil-borne diseases according to the present invention comprises inoculating the roots of plants to be protected with living cells of the *Pseudomonas solanacearum* M4S strain. It should be noted herein that the term "root", referred to in this specification and the appended claims, means a portion which exists in soil and through which water or nutrients are absorbed from the soil when cultivation is conducted by using soil, and that it also includes a potato tuber or the like. Inoculation of the roots can be readily carried out by immersing the roots in a suspension of the bacteria. The suspension may be prepared by suspending the organism at the rate of from $10^6$ to $10^{10}$ and preferably from $10^7$ to $10^9$ cells per liter of an appropriate medium, preferably water. In instances where the plants to be protected are seedlings, the roots of the seedlings to be transplanted to the field may be immersed for a period of time, usually, ranging from 30 minutes to 3 hours, and preferably for about 1 hour. In the case of potatoes, seed potatoes cut for transplanting may be immersed in the above-mentioned suspension for, usually, from 30 minutes to 3 hours, and preferably for approximately 1 hour. The timing of treatment may range from 3 weeks before transplating to, preferably, the day of transplanting.

Plants designated for control of diseases according to the present invention may include solanaceous plants such as tobacco plants, eggplants, tomatoes, green peppers, potatoes or the like, while diseases to be controlled may include bacterial wilt of tobacco and other solanaceous plants including brown rot of potatoes, as caused by the bacteria of *Pseudomonas solanacearum*. The timing of application at the seedling stage prior to transplanting is preferable: usually from 40 to 60 days after seedling for tobacco plants, 60 to 120 days for eggplants, 60 to 80 days for tomatoes and from 60 to 120 days for green peppers. In the case of potatoes, the application should be conducted at, preferably, the time of cutting for transplanting.

*Pseudomonas solanacearum* M4S may be cultured in the same manner as the known parent strain, *Pseudomonas solanacearum*. For instance, it may be cultured by inoculating a CPG medium (1 g of casamino acid, 10 g of glucose, 10 g of peptone and 1,000 ml of water) and shaking it at 28 to 30°C for approximately 48 hours, or, it may be cultured on either slant or plate agar medium.

Acquisition of Pseudomonas solanacearum M4S

1. Isolation of colonies of mutant strains

*Pseudomonas solanacearum* (U-7 strain), which is known as a casual bacterium of bacterial wilt of tobacco, was statically cultured in a test tube with the strain inoculated in a CPG medium at 37°C for 1 week without light. The inoculum was incubated by streaking on a CPG agar culture medium with a sterilized platinum loop. When a line is drawn with a sterilized platinum loop, the concentration of the inoculum to be inoculated around the beginning of the line so high that colonies are formed in dense population, whereas the concentration around the end of the line is so thin as to provide an isolated single colony. This procedure produced 35 isolated colonies.

2. Selection of strains capable of growing well in competition with virulent U-7 strain.

A CPG agar culture medium was prepared and allowed to cool to 45°C at which the agar was just about to solidify. A suspension of *Pseudomonas solanacearum* U-7 strain ($10^9$ cells/ml) was added at the rate of 1 ml per liter of this medium, and the medium was immediately poured, in the amount of 15 ml, into petri dishes with a diameter of 9 cm. After the agar was solidified, the 35 strains obtained previously were cultured on this medium by streaking. After incubation at 27°C for 48 hours, observation of the growing status of each strain revealed that 11 strains had grown well.

3. Selection of avirulent strains

Suspension ($10^9$ cells/ml) of the 11 strains, obtained as described above, was inoculated, by means of leaf-infiltration method, into the 8th to 10th fully expanded leaves from the cotyledon of tobacco plants not less than 10 weeks after seeding. Unlike the U-7 strain, none of the strains caused any wilting of any portion other than the leaves into which the bacterium was inoculated, although most of the strains wilted the whole leaf or the portion of the leaf infiltrated with bacterium. Only one out of the 11 strains did not cause necrosis in any portion except the site of infiltration. This last virulent strain was the *Pseudomonas solanacearum* M4S strain used for the present invention.

Roots of the above-mentioned tobacco plants were immersed in the above suspension of each of the 11 strains for inoculation, and so as to observe the state of disease occurrence. It was found that two of the 11 strains did not cause any disease, and one of the two was the M4S strain employed in the present invention.

4. Selection of strain inhibiting the growth of virulent *Pseudomonas solanacearum* U-7

The 11 strains obtained above were each poured into a TZC plate culture medium in a petri dish with a diameter of 9 cm. Each of the mutants was streaked thereon and the culture medium was incubated at 27°C for 36 hours. A U-7 strain suspension ($10^9$ cells/ml) was added at the rate of 1 ml per liter of the TZC culture medium immediately before the agar was allowed to solidify and both were mixed with each other. The mixture, in the amount of 9 ml, was poured on the TZC medium on which each of the 11 strains grew. Immediately after cooled down, the culture medium was incubated at 27°C for 36 hours. Each of the 11 strains inhibited growth of the U-7 strains on the bi-layer medium. Two of the 11 strains inhibited growth very severely, and one of the two was the M4S strain to be employed in the present invention.

As indicated above, the *Pseudomonas solanacearum* M4S strain to be used in the present invention is an ideal strain having a combination of three properties such that, among the 35 mutants obtained from the virulent *Pseudomonas solanacearum* U-7 strain, it was the least virulent, grew to the best extent in competition with the U-7 strain, and inhibited growth of the U-7 strain to the furthest extent.

## Example 1

Twenty-five tobacco seedlings (cultivar: Shiroenshu No. 1) were grown in polyvinyl chloride pots, having sides of 28.5 cm, so that they developed 9 true leaves and were suitable for transplanting. Each group of the 20 seelings was immersed in the following three different kinds of liquids for 1 hour. Immersion was performed by pouring each of the liquids into treatment layers of 2 cm depth and immersing the roots of the seedlings therein.

(1) A suspension of living cells of the M4S strain (The suspension was prepared by suspending living cells of the M4S strain in sterilized distilled water in a concentration of $10^{10}$ cells/ml) (present invention).

(2) A suspension of heat-killed cells of the M23R strain (The suspension was prepared by suspending living cells of the M23R strain in sterilized water in the concentration of $10^{10}$ cells/ml, sterilizing the suspension by heat treatment at 100°C for 10 minutes, and leaving it to cool to ambient temperature.)

(3) Sterilized distilled water (control).

Each treated tobacco seedling was transplanted and grown in a clay pot having a diameter of 15 cm. Four days after transplanting, an aqueous suspension containing $10^7$ cells/ml of a virulent tobacco wilt bacterium was prepared, and the suspension was poured at a rate of 10 ml per clay pot immediately after the roots of the 60 transplanted seedlings were injured by thrusting a knife into the roots. Disease incidence was observed 10 days after inoculation of the pathogen, and degree of disease severity was rated in 6 levels from 0 to 5 as shown in Table 1 below. The average disease index was calculated by the equation below, and the control rate was calculated according to the equation below. Test results are shown in Table 2 below.

EP 0 172 914 B1

Table 1

| Disease Index | Disease Incidence |
|---|---|
| 0 | No disease symptoms |
| 1 | Portions of a leaf wilted |
| 2 | One to three leaves wilted |
| 3 | All leaves wilted except two to three leaves close to the meristem |
| 4 | All leaves wilted |
| 5 | Dead |

(where N is the number of seelings tested and n0 to n5 are the numbers of seedlings belonging to the respective indexes 0 to 5).

Control rate = (1 — (average disease index in the treated plot)/(average disease index in the control)) × 100.

Table 2

| Treatment | Disease Incidence rate | Average Disease Index | Control Rate |
|---|---|---|---|
| M4S (Living cell plot) | 30% | 0.40 | 86.8% |
| M23R (Heat-killed cell plot) | 80% | 2.20 | 42.1% |
| Control (Distilled water) | 100% | 3.80 | - |

As shown in Table 2 above, it was found that the M4S strain not only produced the best effect on the control of disease incidence, but also exhibited the disease controlling effect better than the heat-killed M23R strain that is the subject matter of the above-mentioned prior Japanese patent application.

Example 2

The test was carried out following the procedures of Example 1, except that the concentration of the M4S strain in the suspension was $10^8$ cells/ml, the period of time for immersion in the treatment solution was 30 minutes, and the test cultivar of tobacco used included also Mito No. 3. The test results are shown in Table 3 (Test cultivar Shiroenshu No. 1) and in Table 4 (Test cultivar: Mito No. 3).

Table 3

| Treatment | Disease Incidence rate | Average Disease Index | Control Rate |
|---|---|---|---|
| M4S (Living cell plot) | 40% | 1.00 | 74.5% |
| M23R (Heat-killed cell plot) | 68% | 1.90 | 51.3% |
| Control (Distilled water) | 80% | 3.90 | - |

5

Table 4

| Treatment | Disease Incidence rate | Average Disease Index | Control Rate |
|---|---|---|---|
| M4S (Living cell plot) | 44% | 1.30 | 67.5% |
| M23R (Heat-killed cell plot) | 72% | 2.80 | 30.0% |
| Control (Distilled water) | 84% | 4.00 | - |

As shown in both Tables 3 and 4, in each case, regardless of the plant cultivars used, the tobacco plant treated with the living cells of the M4S strain indicated the lowest disease incidence rate, the one treated with the heat-killed M23R strain cells gave the next lowest, and the control where the tobacco plant was treated with water showed the highest disease incidence rate. The average disease indexes indicating the severity of disease occurrence were of the same order as in the case of the disease incidence rate. The difference between the treated plot and the control plot is statistically significant (level of significance, 1%), as is the difference between the plot of the heat-killed cells of the M23R strain and the plot of the living cells of the M4S strain (level of significance, 5%). Thus, the control rate was considerably improved through treatment with living cells of the M4S strain.

Example 3

Eggplant seedlings (cultivar: Gunko No. 2) were transplanted twice after seeding in polyvinyl chloride pots and allowed to grow for 90 days after seeding until 8 true leaves had developed and the plants had grown enough to be transplanted. The test was carried out using an M4S strain suspension in the concentration of $10^9$ cells/ml, and a suspension of heat-killed cells of the M23R strain prepared in the same manner as in Example 1. Sterilized distilled water was used for the control plot. After immersion in the liquids, each plot, 20 eggplant seedlings in total, was transplanted into pots with a diameter of 15 cm. After 4 days, the seedlings were treated with a suspension of a virulent eggplant wilt bacteria in the same manner as in Examaple 1. After additional 14 days, disease occurrence was observed and disease incidence rate, average disease index and control rate were calculated in the same manner as in Example 1. The results are shown in Table 5 below.

Table 5

| Treatment | Disease Incidence rate | Average Disease Index | Control Rate |
|---|---|---|---|
| M4S (Living cell plot) | 15% | 0.15 | 96.2% |
| M23R (Heat-killed cell plot) | 40% | 0.75 | 81.0% |
| Control (Distilled water) | 85% | 3.95 | - |

As shown in Table 5 above, the treatment plots were lower in the disease incidence rate than the control plot, and lower than the control plot in the average disease index with a statistical significance (level of significance: 1%). It is also to be noted that the plot treated with living cells of the M4S strain was lower in the disease incidence rate and the average incidence index than the plot treated with heat-killed cells of the M23R strain.

Example 4

Each of the M4S living cells, and living cells of the 305 strain reported as effective for control of bacterial wilt of tobacco heretofore, was suspended in sterilized distilled water in the concentration of $10^9$ cells/ml. Sterilized distilled water was employed as the control. Each plot, 24 tobacco plants in total, was immersed in each of the suspensions for 30 minutes. After immersion, procedures and evaluations were carried out in the same manner as in Example 1. The identical test was repeated twice, and the test results are shown in Table 6 (Test I) and Table 7 (Test II).

## Table 6

| Treatment | Disease In-cidence rate | Average Disease Index | Control Rate |
|---|---|---|---|
| M4S (Living cell plot) | 16.7% | 0.17 | 90.7% |
| 305 (Living cell plot) | 41.7% | 1.58 | 13.7% |
| Control (Dis-tilled water) | 58.3% | 1.83 | - |

## Table 7

| Treatment | Disease In-cidence rate | Average Disease Index | Control Rate |
|---|---|---|---|
| M4S (Living cell plot) | 50.0% | 1.75 | 50.0% |
| 305 (Living cell plot) | 91.7% | 3.75 | -7% |
| Control (Dis-tilled water) | 83.3% | 3.50 | - |

In each case in which two different pathogens were inoculated, the disease incidence rates of tobacco plants treated with the M4S living cells were low, while the disease incidence rates of tobacco plants treated with living cells of the 305 strain were slightly lower in Test I and slightly higher in Test II than the control plot. The average disease indexes were of the same order, and the difference between the control plot and the plot treated with the M4S strain was statistically significant (level of significance: 1%), whereas the difference between the plot treated with the 305 strain and the control plot was not significant statistically.

## Example 5

Tomato seedlings (cultivar: Fukujyo No. 100) grown for 4 weeks after seeding were employed in this test. They were treated in the same manner as in Example 4, with the exception that the number of plants treated with sterile distilled water was 20. After the treatment, the seedlings were planted in clay pots with a diameter of 12 cm and treated, 4 days later, with a suspension of wilt bacteria. After another 10 days, the state of disease incidence was observed, and the disease incidence rates, average disease indexes and control rates were calculated in the same manner as in Example 1. The results are shown in Table 8 below.

## Table 8

| Treatment | Disease In-cidence rate | Average Disease Index | Control Rate |
|---|---|---|---|
| M4S (Living cell plot) | 10.0% | 0.50 | 76.7% |
| 305 (Living cell plot) | 25.0% | 1.15 | 46.5% |
| Control (Dis-tilled water) | 45.0% | 2.15 | - |

It was found that the plots treated were lower than the control plot in both the disease incidence rates and the average disease indexes. In particular, the plot treated with the M4S living cells was lower in the disease incidence rate than the plot treated with the 305 living cells, and was lower in the average disease index with a statistical significance (level of significance: 1%) as compared to the control plot.

Example 6

A total of 54 tobacco seedlings (cultivar: Shiroenshu No. 1), grown in the same manner as in Example 1, were used, per plot, for the test. In the same manner as in Example, 1 the roots of the tobacco seedlings were immersed, for 30 minutes, in a suspension of the M4S living cells at the concentration of $10^{10}$ cells/ml. In the control plot, no treatment was made. Immediately after immersion, the tobacco seedlings were transplanted to two locations in a test field infested with bacterial wilt of tobacco. After transplanting, they were cultivated according to the conventional method of cultivation, harvested and cured. One hundred and eight days after transplanting, investigation of disease incidence was conducted in the same manner as in Example 1. The harvested leaves were divided into grades. Yields and prices per 10 ares were calculated, prices being based on receipts for leaf tobacco in 1983. The results are shown in Tables 9 and 10 below, the yields being in kilograms, and the prices in Japanese currency.

Table 9

|  | Disease Incidence Rate | Average Disease Index | Control Rate | Yield Per 10 Ares | Price |
|---|---|---|---|---|---|
| Treat- ment Plot | 78% | 2.76 | 26.6% | 255.0 | 318,145 |
| Control Plot | 96% | 3.76 | - | 226.7 | 290,664 |

Table 10

|  | Disease Incidence Rate | Average Disease Index | Control Rate | Yield Per 10 Ares | Price |
|---|---|---|---|---|---|
| Treat- ment Plot | 43% | 1.22 | 51.4% | 287.6 | 384,419 |
| Control Plot | 72% | 2.51 | - | 281.6 | 332,946 |

In each case, the plots treated were lower than the control plot both in the disease incidence rate and in the average disease index, with statistical significance (level of significance: 5%). The control rates in the plots treated were 26.6% and 51.4%, respectively.

**Claims**

1. A *Pseudomonas solanacearum* M4S strain (international deposition number FERM BT-700).

2. A composition for control of bacterial wilt of tobacco and other solanaceous plants, containing living cells of a *Pseudomonas solanacearum* M4S (international deposition number FERM BT-700) strain and a pesticidally acceptable carrier.

3. A composition according to claim 2, wherein the composition is a suspension of living cells of the said *Pseudomonas solanacearum* M4S strain in water.

4. A composition according to claim 3, wherein the concentration of the said *Pseudomonas solanacearum* M4S strain in the composition is from $10^6$ to $10^{10}$ cells/ml.

5. A process for controlling bacterial wilt of tobacco and other solanaceous plants which comprises inoculating roots of the solanaceous plants with living cells of a *Pseudomonas solanacearum* M4S strain (international deposition number FERM BT-700).

6. A process according to claim 5, wherein the inoculation is carried out by immersing the roots of the solanaceous plants in a suspension of living cells of the said *Pseudomonas solanacearum* M4S strain.

7. A process according to claim 6, wherein the suspension is a suspension of the living cells of the said *Pseudomonas solanacearum* M4S strain in water in a concentration of from $10^6$ to $10^{10}$ cells/ml.

8. A process according to claim 5, wherein the solanaceous plants are tobacco plants, eggplants, tomato plants, green pepper plants or potato plants.

**Patentansprüche**

1 Pseudomonas solanacearum von Stamm M4S (internationale Hinterlegungsnummer FERM BT-700).

2. Zusammensetzung zur Kontrolle von bakteriell bedingtem Welken von Tabakpflanzen und anderen Nachtschattengewächsen, enthaltend lebende Zellen von Pseudomonas solanacearum vom Stamm M4S (internationale Hinterlegungsnummer FERM BT-700) und einen pestizid-akzeptablen Carrier.

3. Zusammensetzung nach Anspruch 2, wobei diese eine Suspension lebender Zellen des Pseudomonas solanacearum von Stamm M4S in Wasser ist.

4. Zusammensetzung nach Anspruch 3, wobie die Konzentration des Pseudomonas solanacearum von Stamm M4S in der Zusammensetzung $10^6$—$10^{10}$ Zellen/ml beträgt.

5. Verfahren zur Kontrolle von bakteriell bedingtem Welken von Tabakpflanzen und anderen Nachtschattengewächsen, umfasend das Impfen von Wurzeln der Nachtschattengewächse mit lebenden Zellen eines Pseudomonas solanacearum von Stamm M4S (internationale Hinterlegungsnummer FERM BT-700).

6. Verfahren nach Anspruch 5, wobei das Impfen durch Eintauchen der Wurzeln der Nachtschattengewächse in eine Suspension lebender Zellen des Pseudomonas solanacearum von Stamm M4S erfolgt.

7. Verfahren nach Anspruch 6, wobei die Suspension eine Suspension der lebenden Zellen des Pseudomonas solanacearum von Stamm M4S in Wasser in einer Konzentration von $10^6$—$10^{10}$ Zellen/ml ist.

8. Verfahren nach Anspruch 5, wobei die Nachtschattengewächse Tabak-, Eierfrucht-, Tomaten-, grüne Paprika- oder Kartoffel- pflanzen sind.

**Revendications**

1. Souche *Pseudomonas solanacearum* M4S (numéro de dépôt international FERM BT-700).

2. Composition pour lutter contre le flétrissement bactérien du tabac et d'autres plantes solanacées, qui contient des cellules vivantes d'une souche *Pseudomonas solanacearum* M4S (numero international de dépôt FERM BT-700) et un véhicule acceptable dans le domaine des pesticides.

3. Composition conforme à la revendication 2, qui est une suspension de cellules vivantes de ladite souche *Pseudomonas solanacearum* M4S dans de l'eau.

4. Composition conforme à la revendication 3, dans laquelle la concentration de ladite souche *Pseudomonas solanacearum* M4S dans la composition vaut de $10^6$ à $10^{10}$ cellules/ml.

5. Procédé de lutte contre le flétrissement bactérien du tabac et d'autres plantes solanacées, qui comporte l'inoculation, à des racines de plants de solanacées, de cellules vivantes d'une souche *Pseudomonas solanacearum* M4S (numéro international de dépôt FERM BT-700).

6. Procédé conforme à la revendication 5, dans lequel l'inoculation est effectuée par immersion des racines des plants de solanacées dans une suspension de cellules vivantes de ladite souche *Pseudomonas solanacearum* M4S.

7. Procédé conforme à la revendication 6, dans lequel la suspension est une suspension de cellules vivantes de ladite souche *Pseudomonas solanacearum* M4S dans de l'eau en une concentration valant de $10^6$ à $10^{10}$ cellules/ml.

8. Procédé conforme à la revendication 5, dans lequel les plants de solanacées sont des plants de tabac, d'aubergine, de tomate, de poivre vert ou de pomme de terre.